# EUROPÄISCHE PATENTSCHRIFT

(11) **EP 0 946 149 B1**
(45) Veröffentlichungstag und Bekanntmachung des Hinweises auf die Patenterteilung: **19.06.2002**
(21) Anmeldenummer: 97953625.7
(22) Anmeldetag: 15.12.1997
(51) Int. Cl.: A61K 9/14, A61K 9/16, A61K 9/20

(54) **HOMOGENE, STEROIDE IN HOHER KONZENTRATION ENTHALTENDE, PRÄFORMULIERUNGEN ZUR HERSTELLUNG NIEDRIGDOSIERTER FESTER UND HALBFESTER PHARMAZEUTISCHER ZUBEREITUNGEN**
HOMOGENEOUS PREFORMULATIONS CONTAINING HIGH CONCENTRATIONS OF STEROIDS, FOR PRODUCING LOW-DOSE SOLID AND SEMI-SOLID PHARMACEUTICAL PREPARATIONS
PREFORMULATIONS HOMOGENES, A FORTE CONCENTRATION EN STEROIDES, SERVANT A PRODUIRE DES PREPARATIONS PHARMACEUTIQUES SOLIDES ET SEMI-SOLIDES FAIBLEMENT DOSEES

(30) Priorität: 16.12.1996 DE 1965219
(43) Veröffentlichungstag der Anmeldung: 06.10.1999
(73) Patentinhaber: JENAPHARM GmbH, D-07745 Jena (DE)
(72) Erfinder: GRAWE, Detlef, D-99510 Kleinromstedt (DE); HÖSEL, Peter, D-07745 Jena (DE); MÖLLMANN, Peter, D-07749 Jena (DE); TIMPE, Carsten, D-37299 Weissenborn (DE); DITTGEN, Michael, D-99510 Apolda (DE); MATTHEY, Klaus, D-07745 Jena (DE)
(74) Vertreter: Cramer, Eva-Maria
(86) Internationale Anmeldenummer: DE9702915
(87) Internationale Veröffentlichungsnummer: WO9826762

(56) Entgegenhaltungen:
- EP-A- 0 606 486
- WO-A-93/12797
- DE-A- 4 426 709

## Beschreibung

Die Erfindung betrifft homogene, Steroide in hoher Konzentration enthaltende, Präformulierungen zur Herstellung niedrigdosierter fester und halbfester pharmazeutischer Zubereitungen mit einem Konzentrationsgehalt von 0,001 bis 1 Steroid-Gewichtsprozent.
Weiterhin betrifft die Erfindung ein Verfahren zur Herstellung der homogenen, Steroide in hoher Konzentration enthaltenden, Präformulierung für die angesprochene niedrigdosierte feste und halbfeste pharmazeutische Zubereitung.

Es ist bekannt, daß für die zuverlässige Wirkung eines Arzneimittels die homogene Wirkstoffverteilung eine wesentliche Voraussetzung ist.
Das Problem der homogenen Wirkstoffverteilung tritt besonders bei einzeldosierten festen und halbfesten Zubereitungsformen auf, in denen der Wirkstoff nicht in gelöster Form vorliegt, beispielsweise Tabletten, Fragees, Kapseln und Suppositorien.
Voraussetzung für die homogene Wirkstoffverteilung in den einzeldosierten Arzneiformen ist einerseits eine gleichmäßige Verteilung des Wirkstoffes in der Grundmischung bzw. Präformulierung und andererseits das Beibehalten dieser Verteilung während der weiteren Verarbeitung. Inhomogenitäten in der Wirkstoff-Hilfstoffmischung und Ungenauigkeiten beider Weiterverarbeitung überlagern sich und führen zu Dosierungsschwankungen in festen und halbfesten einzeldosierten Arzneiformen.
Bei hochdosierten Zubereitungen treten in der Regel keine gravierenden Mischungsinhomogenitäten auf.
Bei niedrigdosierten Arzneiformen besteht das primäre Problem in der Bereitstellung einer ausreichend homogenen und gegen Entmischung stabilen Verteilung des im Verhältnis zur Masse der pharmazeutischen Hilfsstoffe sehr geringen Wirkstoffmasse.

Aus der Fach- und Patentliteratur sind niedrigdosierte feste und halbfeste steroidhaltige Zubereitungen bekannt, wobei die Herstellung der Präformulierungen mittels trockner und feuchter Granuliertechnologien oder trockenen Mixingverfahren erfolgt.
M. DITTGEN, H. KALA et al. beschreiben beispielsweise in "Zur pharmazeutischen pharmazeutischen Technologie der Granulierung", Pharmazie 35, H.4, S. 237-249, 1980 derartige Granuliertechnologien.
Problematisch dabei ist, eine gute Gleichförmigkeit (content uniformity) unter Vermeidung von Entmischungs- und Klassiereffekten, z.B. durch Abwirbeln und Ausblasen von feinen Wirkstoffpartikeln aus der Wirbelschicht, durch den Mischvorgang zu erreichen.
Nachteilig an nach diesen Technologien hergestellten Formulierungen ist
- der Einsatz mikronisierter Wirkstoffe und damit verbunden die Notwendigkeit einer zusätzlichen Prozeßstufe,
- ableitend daraus, die Möglichkeit der Rekristailisation und Agglomeratbildung über die aktiven Flächen der Wirkstoffpartikel und damit verbunden die negativen Auswirkungen auf Homogenität und Freisetzungseigenschaften.

In einer speziellen Form der Wirbelschicht-Sprühgranulierverfahren wird der Einsatz mikronisierter Wirkstoffe umgangen, indem der Wirkstoff in einem organischen Lösungsmittel in hoher Verdünnung gelöst und auf einen in einer Wirbelschicht bewegten grobkörnigen Hilfstoff aufgesprüht und aufbauend granuliert wird .
Die Nachteile der nach diesem Verfahren hergestellten Formulierungen sind:
- Entstehen von Abweichungen vom gewünschten Steroidgehalt durch Ausblasen von feinem steroidalen Abrieb aus der Wirbelschicht,
- daß aufgrund der langen Verweilzeiten im Herstellungsprozess es bei thermisch instabilen Wirkstoffen zu unerwünschten Zerfallsprozessen kommen kann.

Ein nicht zu unterschätzender Nachteil des Verfahrens besteht darin, daß großen Mengen Lösungsmittel in den Finalstufen der galenischen Zubereitung werden, wobei es eines hohenr anlagen- und sicherheitstechnischen Aufwandes (Ex-Schutz verbunden mit Stickstoffinertisierung) bedarf.

In der EP 0 503 521 wird beschrieben, daß eine direkttablettierbare pharmazeutische Zubereitung durch Trockenmischung mikronisierter Steroide mit bestimmten Exzipienten (sprühgetrockneter Lactose) erreich wird.
Es erweist sich als nachteilig:
- Nur ausgewählte Exzipienten weisen die erforderlichen adsorptiven Eigenschaften auf,
- Die Beladungskapazität der Oberflächen ist nur sehr begrenzt,
- Es müssen ebenfalls mikronisierte Wirkstoffe verwendet werden, die den Nachteil der Rekristallisation und Agglomeratbildung über die aktiven Flächen der Wirkstoffpartikel und damit verbunden die negativen Auswirkungen auf Homogenität und Freisetzungseigenschaften aufweisen.

M. DEKKER beschreibt in 'The spray drying of pharmaceuticals', Drug Development an Industrial Pharmacy, 18 (11&12), S. 1169-1206, 1992 und
E. NÜRNBERG in ' Darstellung und Eigenschaften pharmazeutisch relevanter Sprühtrocknungsprodukte', Acta Pharmaceutica Technologica, 26 (1), S. 40-67, 1980
den Einsatz der Sprühtrocknungstechnologie zur gaienischen Formulierung pharmazeutischer Wirkstoffe, darunter auch Steroide.
Mit der Sprühtrocknung werden in erster Linie über Wirkstoffeinbettungen bzw. Mikroverkapselungen (z.B. in polymere Hilfstoffe) oder Einschlußkomplexe (in Cyclodextrine) retardierende oder löslichkeits- und stabilitätsverbessernde Wirkungen erzielt.

Nach J. COOPER; und J.E. REES, 'Tableting research and technology', J.Pharm.Sci. 61 (1972),S. 1551-1555. kann die Sprühtrocknung eingesetzt werden
- zum Überziehen von Arzeineistoffpartikeln mit schützenden Hüllen und zur Modifikation der physikalischen Eigenschaften der Wirkstoffe und Hilfstoffe mit dem Ziel ihre Weiterverarbeitung (z.B. Tabletten) zu erleichtern.
Nach H. KALA et al., 'The use of spraydrying in pharmacy', Pharmazie, 34, H. 12(1979), 779-784 ist der Einsatz der Sprühtrocknung zur Erzielung bestimmter Eigenschaften bei Wirk-und Hilfsstoffen, die über eine einfache Trocknung und gleichzeitige Mikronisierung hinausgehen, grundsätzlich bekannt.
Beispielsweise können metastabile Arzneistoffmodifikationen bzw. amorphe Wirkstoffe standardisiert hergestellt werden, um die Auflösungsgeschwindigkeit zu verbessern oder Unsicherheitsfaktoren auszuschalten, die durch die Existenz verschiedener Kristallmodifikationen bedingt sind.
In diesem Zusammenhang werden insbesondere Sprüheinbettungen in verschiedene Hilfstoffe und Sprühtrocknung von Suspensionen aus Arzneistofflösung und unlöslichem Hilfsstoff (Cellulosen) erwähnt.

Außerdem erwähnen H. KALA et al., daß bereits Sprühprodukte (Hilfsstoffe als Trockenbindemittel , Wirkstoffe und Kombinationen) in Gestalt von kugelförmigen, frei fließenden Partikeln für die Direkttablettierung zum Einsatz kamen.

Auch M. DEKKER erwähnt in 'The spray drying of pharmaceuticals', Drug Development an industrial Pharmacy, 18 (11&12), S. 1169-1206, 1992, daß feine freifließende Hilfsstoffgranulate (Füllstoffe, Binder, Sprengmittel, Farbstoffe) für die Direkttablettierung über Sprühtrocknung hergestellt werden können.
Zweck dieser Sprühgranulierverfahren ist es, günstigere Verarbeitungseigenschaften, die jedoch in der Regel mit einer Kornvergröberung verbunden sind, für die Direkttablettierung zu erzielen.

Der Nachteil dieser Technologien hinsichtlich der direkten Herstellung von niedrigdosierten pharmazeutischen Zubereitungen besteht darin:
- Aufgrund des groben Korns dieser Granulate ist die erforderliche Gehaltsgleichförmigkeit über ein nachfolgendes Mixing nicht immer gegeben,
- die Sprühgranulierung der notwendigerweise hochverdünnten Steroid-Lösungen bzw. -Suspensionen ist wegen der großen Volu mina sehr aufwendig.

Homogene, Steroide in hoher Konzentration enthaltende, Präformulierungen zur Herstellung niedrigdosierter fester und halbfester pharmazeutischer Zubereitungen mit einem Konzentrationsgehalt von 0,001 bis 1 Steroid-Gewichtsprozent und die Herstellung einer homogenen, Steroide in hoher Konzentration enthaltenden, Präformulierung für eine niedrigdosierte feste und halbfeste pharmazeutische Zubereitung mit einem Konzentrationsgehalt von 0,001 bis 1 Steroid-Gewichtsprozent mittels Sprühtrocknung, wobei in einem konventionellen Trockenmischverfahren unter Vermeidung der o.g. Nachteile die Präformulierung zu einer direkttablettierbaren pharmazeutischen Zubereitung verarbeitet werden kann, sind in der Patent- und Fachliteratur nicht beschrieben.

Der Erfindung liegt die Aufgabe zugrunde, steroidhaltige Präformulierungen bereitzustellen, die die Herstellung niedrigdosierter fester und halbfester pharmazeutischer Zubereitungen mit einem Konzentrationsgehalt von 0,001 bis 1 Steroid-Gewichtsprozent mit guter Homogenität des Steroid-Hilfsstoffgemisches verbunden mit einer guten Stabilität der Wirkstoffverteilung in der pharmazeutischen Zubereitung gewährleisten.

Die Aufgabe wird erfindungsgemäß durch die Bereitstellung homogener, Steroide in hoher Konzentration enthaltender, Präformulierungen zur Herstellung niedrigdosierter fester und halbfester pharmazeutischer Zubereitungen mit einem Konzentrationsgehalt von 0,001 bis 1 Steroid-Gewichtsprozent erhältlich durch Verdampfen des den steroidalen Wirkstoff enthaltenden Dispersionsmittels aus einer mit pharmazeutisch üblichen Hilfsstoffen versetzten Suspension, wobei man einen Sprühnebel erzeugt, dessen mittlerer Tropfendurchmesser größer ist als der mittlere Durchmesser der Hilfstoffpartikel und die maximale Korngröße der getrockneten Partikel der steroidhaltigen Präformulierung um weniger als 30 % im Verleich zur Korngröße des Hilfsstoffes vergrößert, gelöst.

Vorteilhafte Ausgestaltungen der Erfindung werden in den Ansprüchen 2 und 3 aufgezeigt.

Es erweist sich als besonders vorteilhaft, wenn man die maximale Korngröße der getrockneten Partikel der steroidhaltigen Präformulierung um weniger als 10 % im Vergleich zur Korngröße des Hilfsstoffes vergrößert.

Auch ist besonders vorteilhaft daß die Konzentration des steroidalen Wirkstoffes in der steroidhaltigen Präformulierung 5-30 Gewichtsprozent beträgt.

Eine weitere vorteilhafte Ausgestaltung der Erfindung ist im Anspruch 4 aufgezeigt.

Anspruch 5 beschreibt das erfindungsgemäße Verfahren zur Herstellung einer homogenen, Steroide in hoher Konzentration enthaltenden, Präformulierung für eine niedrigdosierte feste und halbfeste pharmazeutische Zubereitung mit einem Konzentrationsgehalt von 0,001 bis 1 Steroid-Gewichtsprozent.

Die vorteilhafte Ausgestaltungen des erfindungsgemäßen Verfahrens wird im Anspruch 6 aufgezeigt.

Als Sexualhormon kommt vorzugsweise ein Bestandteil aus der Gruppe der Estrogene, der Gestagene und der Antigestagene oder ein Bestandteil, der die vorgenannten Wirkstoffe nach Verabreichung rasch abspaltet, zum Einsatz.

Im folgenden wird das erfindungsgemäße Verfahren zur Herstellung der erfindungsgemäßen homogenen, Steroide in hoher Konzentration enthaltenden, Präformulierungen näher beschrieben werden:
Der oder die steroidalen Wirkstoffe werden in einem geeigneten Lösungsmittel, z.B. Ethanol, gelöst.
Es erfolgt eine eventuelle Zugabe von stabilitätsverbessernden, resorptionsbeschleunigenden oder die Freisetzungskinetik modifizierenden Hilfsstoffen. Anschließend wird der Hilfsstoff mit einem Masseverhältnis zum gelösten Steroid von 1:1 bis 100000:1 suspendiert. Dabei erweist sich ein Bereich von 2:1 bis 20:1 für das Verfahren als besonders vorteilhaft. Als Hilfsstoffe können z.B. α-Lactosemonohydrat, mikrokristalline Cellulose oder anorganische Calziumsalze, vorzugsweise in mikronisierter Form, verwendet werden.
Anschließend erfolgt die Sprühtrocknung der Suspension zur schonenden und raschen Entfernung des Lösungsmittels und zur stabilen und feindispersen Verteilung des Wirkstoffs auf der Hilfsstoffoberfläche.
Es ist besonders bemerkenswert, daß aus der versprühten Suspension Wirkstoff- und Hilfsstoffpartikel nicht nebeneinander getrocknet werden. Durch Wahl der Prozeßbedingungen beim Sprühvorgang kann der Wirkstoff nahezu vollständig und stoffschlüssig auf dem Hilfsstoff gebunden werden kann.
Eine wesentliche Rolle spielen dabei die Korngößenverteilung des Hilfsstoffs, die Feststoffkonzentration der Suspension, die Wirkstoffkonzentration der Lösung und die Tropfengrößenverteilung im Sprühnebel, die wiederum von der Wirkung des Zerstäuberaggregates (Düse, Scheibe) und von der Viskosität der Lösung wesentlich beeinflußt wird.
Der Sprühprozeß ist so zu fahren, daß der mittlere Tropfendurchmesser im Sprühnebel größer ist als der mittlere Durchmesser der Hilfsstoffpartikel, aber auch nicht so groß, daß durch Granulierung eine wesentliche Kornvergröberung eintritt.
In Abhängigkeit vom verwendeten Sprühtrocknungsgerät und vom Stoffsystem sind die Prozeßbedingungen spezifisch einzustellen.

Die so hergestellten steroidalen Präformulierungen werden mit anderen direkttablettierbaren Hilfsstoffen, wie z.B. Tablettose , Ludipress, Emcompress oder Avicel in einem geeigneten Mischer trocken gemischt und anschließend in Hartgelatine- oder als Suspension in öliger Form in Weichgelatine-Kapseln abgefüllt oder in üblicher Form tablettiert. Die Tabletten werden mit den üblichen und bekannten Hilfsstoffen dragiert oder wäßrig oder organisch beschichtet.

Mit der Erfindung werden homogene, Steroide in hoher Konzentration enthaltende, Präformulierungen zur Herstellung niedrigdosierter fester und halbfester pharmazeutischer Zubereitungen mit einem Konzentrationsgehalt von 0,001 bis 1 Steroid-Gewichtsprozent bereitgestellt, die sich durch ihre Homogenität und Stabilität gegen Entmischung auszeichnen, wobei der steroidale Wirkstoff feindispers und mit dem Hilfsstoff stoffschlüssig verbunden enthalten ist.
Es wurde überraschenderweise gefunden, daß derartige steroidale Präformulierungen in einem geeigneten Mischer trocken mit den üblichen Tablettierhilfsstoffen problemlos zu niedrigdosierten pharmazeutischen Zubereitungen mit sehr guter und stabiler Wirkstoffhomogenität verarbeitet werden können.

Die Prüfung auf Gleichförmigkeit des Gehaltes der einzeldosierten Arzneiformen erfolgt nach den Vorschriften der PhEur und des DAB 9.
Sie beruht auf der Bestimmung des Arzneistoffgehaltes einer Anzahl einzeldosierter Einheiten, um festzustellen, ob der Einzelgehalt innerhalb der festgesetzten Grenzen liegt, bezogen auf den Durschschnittsgehalt eines Musters.

### Ausführung:

In 10 willkürlich nach dem Stichprobenverfahren entnommenen Einheiten wird einzeln der Arzneistoffgehalt mit Hilfe eines geeigneten analytischen Verfahrens bestimmt.

Tabelle 1 zeigt die Eigenschaften von Tabletten auf, deren Präformulierung nach Ausführungsbeispiel 2 hergestellt wird.

| *Tabletten mit 30 µg Ethinylestradiol* | |
|---|---|
| Gleichförmigkeit des Gehaltes nach Ph.Eur./DAB: | 29 µg |
| relative Standardabweichung - S rel : | 2.26 % |
| Entsprechend Content Uniformity Test - anzuwenden bei einem Wirkstoffanteil von 50 mg und weniger pro Einzeldosis- wird die Homogenität bewiesen. | |
| | |
| Es wird außerdem nachgewiesen: | |
| Wirkstoff-Freisetzung (n=6) | 82% nach 45 min |
| | Forderung: >= 75% nach 45 min |
| S rel : | 1.53% |
| | |
| Härte (n=10): | 35,6 N |
| Gleichförmigkeit der Tablettenmasse nach Ph.Eur./DAB: | erfüllt |
| mittlere Tablettenmasse (n=100): | 50,7 mg |
| S rel: | 2.10 % |
| max: | 54,0 mg |
| min: | 49,0 mg |

Tabelle 2 zeigt die Eigenschaften von Tabletten auf, deren Präformulierung nach Ausführungsbeispiel 3 hergestellt wird.

| *Tabletten mit 250 µg Sulfamatsteroid* | |
|---|---|
| Gleichförmigkeit des Gehaltes nach Ph.Eur./DAB: | 253 µg |
| relative Standardabweichung - S rel : | 1,28 % |
| Entsprechend Content Uniformity Test - anzuwenden bei einem Wirkstoffanteil von 50 mg und weniger pro Einzeldosis- wird die Homogenität bewiesen. | |
| | |
| Es wird außerdem nachgewiesen: | |
| Wirkstoff-Freisetzung (n=6) | 101,0% nach 45 min |
| | Forderung: >= 75% nach 45 min |
| S rel : | 3.4% |
| | |
| Zerfallszeit: | 1:19 min |
| Härte (n=10): | 36,7 N |
| Gleichförmigkeit der Tablettenmasse nach Ph.Eur./DAB: | erfüllt |
| mittlere Tablettenmasse (n=100): | 51,9 mg |
| S rel: | 0.86 % |
| max: | 53,0 mg |
| min: | 51,0 mg |

Tabelle 1 und Tabelle 2 zeigen auf , daß die erfindungsgemäßen steroidalen Präformulierungen zu niedrigdosierten pharmazeutischen Zubereitungen mit sehr guter und stabiler Wirkstoffhomogenität verarbeitet werden können.

Die Vorteile des erfindungsgemäße Verfahren zur Herstellung der erfindungsgemäßen homogenen, Steroide in hoher Konzentration enthaltenden, Präformulierungen sind:
- Sehr gute und stabile Wirkstoffhomogenität in der pharmazeutischen Zubereitung zur Tablettierung.
- Durch die Wahl der Hilfsstoffkörnung wird die für nachfolgende Mischvorgänge wichtige Grenzkorngröße der Präformulierung definiert, da durch das Aufsprühen des Wirkstoffs diese Körnung nur unwesentlich vergröbert wird (siehe Beispiel 1-3, Tabellen 3-5).
- Somit werden Inhomogenitäten und Entmischungs- bzw. Klassiereffekte während des nachfolgenden Mischvorgangs vermieden.
- Die vorteilhaften galenischen Eigenschaften des Hilfsstoffes werden auf den Präformulierung übertragen und die Feststoffeigenschaften des Wirkstoffes werden stabilisiert.
- Es treten keine, wie bei mikronisierten Steroiden häufig beobachtet, Agglomerisationen und damit verbundenen Aktivitätsänderungen auf.
- Es kann auf den Einsatz mikronisierter Steroide verzichtet werden. Dies bedeutet die Einsparung einer aufwendigen Prozeßstufe.
   Mikronisierte Steroidwirkstoffe, wie z.B. Desogestrel, neigen oftmals zu nachträglichen Rekristallisations- und Agglomerisationseffekten, wodurch die Wirkstoffhomogenität und Freisetzung negativ beeinflußt werden.
- Gleichfalls wird der Einfluß der Kristallisation bei der Wirkstoffherstellung, d.h. die oftmals komplizierte Definition und Validierung der Kristallstruktur und Kristallinität, nivelliert.
- Die Präformulierungen sind gegen Entmischung stabil und lagerfähig.
- Durch die Sprühtrocknung der über eine Düse oder Zerstäuberscheibe in einem Trocknungsgas feinverteilten Suspensionstropfen (mit Wirkstofflösung umhüllte Hilfsstoffpartikel) wird durch rasche Abdampfung des Lösungsmittels eine stoffschlüssige und feindisperse Bindung des Wirkstoffs mit der Hilfsstoffoberfläche erreicht. Hierdurch sind die adsorptiven Eigenschaften des Hilfsstoffs, die nach dem Stand der Technik für ein trockenes Mixing mit mikronisierten Steroide entscheidend sind und wodurch die Hilfsstoffauswahl wesentlich eingeschränkt wird, nur von untergeordneter Bedeutung.
- Die Beladung des Hilfsstoffs mit Wirkstoffen kann wesentlich höher sein als bei einem mechanischen Mixing des Hilfsstoffs mit mikronisierten Steroiden.
   Es kann eine Beladung von 1 (g Steroid/g Hilfsstoff) erreicht werden.
   Die erzielbare Beladung ist nur abhängig von der Löslichkeit des Steroids im verwendeten Lösungsmittel, von der Tropfengröße (Sprühbedingungen) und der Teilchengröße des Hilfsstoffs.
- Gemeinsam mit dem gelösten Steroid und suspendiertem Hilfsstoff können weitere stabilitätsverbessernde, resorptionsbeschleunigende oder die Freisetzungskinetik modifizierende Hilfsstoffe, die in gelöster oder suspendierter Form vorliegen können, sprühgetrocknet werden.
- Das Einbringen dieser Hilfsstoffe bereits in der Präformulierungsstufe entlastet die Mixingstufe der pharmazeutischen Endformulierung und überträgt die o.g. Vorteile der Sprühtrocknung bezüglich der Steroidverteilung auf dem Hilfsstoff auch auf diese Hilfstoffe.
- Es ist außerdem möglich, z.B. durch Einsatz entsprechender löslicher Hilfsstoffe den Wirkstoff in der Hilfsstoffhülle molekulardispers einzubetten und somit die Freisetzung zu beschleunigen oder zu retardieren bzw. den Wirkstoff gegen die Umgebung zu schützen.
- Über das Sprühverfahren sind somit mehr galenische Möglichkeiten gegeben als über das trockene Mixing von mikronisierten Steroiden und Hilfsstoffen.
- Das Sprühtrocknungsverfahren ist , da die Abdampfung des Lösungsmittels innerhalb von Sekundenbruchteilen erfolgt, gerade bei thermisch empfindlichen Steroiden (z.B. Sulfamatsteroide) ein äußerst schonendes Verfahren.
- Die o.g. Vorteile erlauben die flexible Herstellung von steroidalen Präformulierungen mit spezifischen galenischen Eigenschaften als zur pharmazeutischen Endformulierung der Tablettenmasse.
- Der galenische Aufwand und die pharmazeutische Technologie zur Endformulierung werden drastisch reduziert und vereinfacht.
- Die in einer Vorstufe hergestellten, mit Steroid hochbeladenen und damit verbunden mengenmäßig geringe Präformulierungen können im trockenen Mixingverfahren problemlos mit den üblichen Tablettierhilfstoffen zu sehr homogenen Tabletten verarbeitet werden.

Die Herstellung der erfindungsgemäßen Präformulierungen soll an den nachfolgenden Beispielen näher erläutert, jedoch dadurch nicht eingeschränkt werden.

### Beispiel 1

25g Desogestrel werden in 562 g Ethanol(96%, DAB) und 63 g Wasser gelöst.
Zur klaren Lösung gibt man unter Rühren 125 g Lactose-Monohydrat (Sorbolac 400, MEGGLE).
Die Suspension wird über einen Sprühtrockner mit Zweistoffdüse im Gleichstromverfahren getrocknet

| | | |
|---|---|---|
| Bedingungen | Lufteintrittstemperatur | 115°C |
| | Luftaustrittstemperatur | 66°C |
| | Zulauf der Suspension | 10 ml/min |
| | Sprühdruck (N2) | 2.5 bar (ü) |
| | Düse | 0.7 mm |
| | Trocknungsluft | 350 L/min |

Das Trockenprodukt wird über einen Zyklon abgeschieden. Korngrößenanalyse:

| | **eingesetzte Lactose** **µm** | **Präformulierung** **µm** |
|---|---|---|
| x10 | 1.57 | 3.37 |
| x50 | 7.94 | 10.76 |
| x90 | 24.18 | 26.75 |

Die Angaben betreffen Aussagen zur maximalen Korngröße.
Beispielsweise stellt die Aussage x90 dar, daß 90 % der eingesetzten Lactose und der Präformulierung in ihrer Korngröße kleiner sind als 24,18 bzw 26,75µm.

### Beispiel 2

12g Ethinylestradiol werden in 180 g Ethanol(96%, DAB) gelöst.
Zur klaren Lösung gibt man unter Rühren 48 g Lactose-Monohydrat (Sorbolac 400, MEGGLE).
Die Suspension wird über einen Sprühtrockner mit Zweistoffdüse im Gleichstromverfahren getrocknet

| | | |
|---|---|---|
| Bedingungen | Lufteintrittstemperatur | 140°C |
| | Luftaustrittstemperatur | 74°C |
| | Zulauf der Suspension | 10 ml/min |
| | Sprühdruck (N2) | 1.3 bar (ü) |
| | Düse | 0.7 mm |
| | Trocknungsluft | 300 L/min |

Das Trockenprodukt wird über einen Zyklon abgeschieden.

### Korngrößenanalyse:

| | **eingesetzte Lactose** **µm** | **Präformulierung** **µm** |
|---|---|---|
| x10 | 1.34 | 1.69 |
| x50 | 6.10 | 7.05 |
| x90 | 18.02 | 20.36 |

### Beispiel 3

6g eines Sulfamatsteroids werden in 240 g Ethanol(96%, DAB) gelöst.
Zur klaren Lösung gibt man unter Rühren 22 g Lactose-Monohydrat (Sorbolac 400, MEGGLE).
Die Suspension wird über einen Sprühtrockner mit Zweistoffdüse im Gleichstromverfahren getrocknet.

| | | |
|---|---|---|
| Bedingungen | Lufteintrittstemperatur | 120°C |
| | Luftaustrittstemperatur | 62°C |
| | Zulauf der Suspension | 10 ml/min |
| | Sprühdruck (N2) | 1.3 bar (ü) |
| | Düse | 0.7 mm |
| | Trocknungsluft | 300 L/min |

Das Trockenprodukt wird über einen Zyklon abgeschieden.

### Korngrößenanalyse:

| | **eingesetzte Lactose** **µm** | **Präformulierung** **µm** |
|---|---|---|
| x10 | 1.34 | 1.43 |
| x50 | 6.10 | 6.40 |
| x90 | 18.02 | 19.86 |

Die Korngrößenanalysen zeigen auf, daß durch die Wahl der Hilfsstoffkörnung die für nachfolgende Mischvorgänge wichtige Grenzkorngröße der Präformulierung definiert wird, da durch das Aufsprühen des Wirkstoffs diese Körnung nur unwesentlich vergröbert wird.
Somit werden Inhomogenitäten und Entmischungs- bzw. Klassiereffekte während des nachfolgenden Mischvorgangs vermieden.

## Patentansprüche

1. Homogene, Steroide in hoher Konzentration enthaltende, Präformulierungen zur Herstellung niedrigdosierter fester und halbfester pharmazeutischer Zubereitungen mit einem Konzentrationsgehalt von 0,001 bis 1 Steroid-Gewichtsprozent erhältlich durch
Verdampfen des den steroidalen Wirkstoff enthaltenden Dispersionsmittels aus einer Suspension mit dispergiertem pharmazeutisch üblichen Hilfsstoff,
wobei man
einen Sprühnebelerzeugt, dessen mittlerer Tropfendurchmesser größer ist als der mittlere Durchmesser der Hilfstoffpartikel
und die maximale Korngröße der getrockneten Partikel der steroidhaltigen Präformulierung um weniger als 30 %
im Vergleich zur Korngröße des Hilfsstoffes vergrößert

2. Homogene, Steroide in hoher Konzentration enthaltende, Präformulierungen zur Herstellung niedrigdosierter fester und halbfester pharmazeutischer Zubereitungen nach Anspruch 1, **dadurch gekennzeichnet,**
**daß** die Konzentration des steroidalen Wirkstoffes 0,001 bis 50 Gewichtsprozent beträgt.

3. Homogene, Steroide in hoher Konzentration enthaltende, Präformulierungen
nach den Ansprüchen 1 und 2, **dadurch gekennzeichnet, daß** der pharmazeutisch übliche Hilfsstoff in mikronisierter Form vorliegt.

4. Homogene, Steroide in hoher Konzentration enthaltende, Präformulierungen
nach den Ansprüchen 1 bis 3, **dadurch gekennzeichnet, daß**
der steroidale Wirkstoff mindestens ein Bestandteil aus der Gruppe der Sexualhormone
darstellt.

5. Verfahren zur Herstellung homogener, Steroide in hoher Konzentration enthaltende, Präformulierungen für niedrigdosierte feste und hafbfeste pharmazeutische Zubereitungen mit einem Konzentrationsgehalt von 0,001 bis 1 Steroid-Gewichtsprozent, **dadurch gekennzeichnet,**
**daß** man aus einer Suspension mit dispergiertem pharmazeutisch üblichen Hilfsstoff das den steroidalen Wirkstoff enthaltende Dispersionsmittel verdampft,
wobei man einen Sprühnebel erzeugt, dessen Tropfendurchmesser größer ist als der mittlere Durchmesser der Hilfsstoffpartikel
und die maximale Korngröße der getrockneten Partikel der steroidhaltigen Präformulierung um weniger als 30 % im Vergleich zur Korngröße des Hilfsstoffes vergrößert.

6. Verfahren zur Herstellung homogener, Steroide in hoher Konzentration enthaltende, Präformulierungen nach Anspruch 5, **dadurch gekennzeichnet, daß** der pharmazeutisch übliche Hilfsstoff in mikronisierter Form vorliegt.

## Claims

1. Homogeneous preformulations which contain steroids in high concentrations for producing low-dose solid and semisolid pharmaceutical preparations with a concentration content of from 0.001 to 1 steroid per cent by weight obtainable by
evaporating the dispersant containing the steroidal active ingredient from a suspension with dispersed pharmaceutically customary excipient,
where a spray mist is generated with an average droplet diameter greater than the average diameter of the excipient particle
and the maximum particle size of the dried particles of the steroid-containing preformulation is increased by less than 30% compared with the particle size of the excipient.

2. Homogeneous preformulations which contain steroids in high concentration for producing low-dose solid and semisolid pharmaceutical preparations according to Claim 1, **characterized in that** the concentration of the steroidal active ingredient is from 0.001 to 50 per cent by weight.

3. Homogeneous preformulations which contain steroids in high concentration according to Claims 1 and 2, **characterized in that** the pharmaceutically customary excipient is present in micronized form.

4. Homogeneous preformulations which contain steroids in high concentration according to Claims 1 to 3, **characterized in that** the steroidal active ingredient represents at least one constituent from the group of sex hormones.

5. Process for producing homogeneous preformulations which contain steroids in high concentration for low-dose solid and semisolid pharmaceutical preparations with a concentration content of from 0.001 to 1 steroid per cent by weight, **characterized in that**
a dispersant containing the steroidal active ingredient is evaporated from a suspension with dispersed pharmaceutically customary excipient,
where a spray mist is generated with an average droplet diameter greater than the average diameter of the excipient particle
and the maximum particle size of the dried particles of the steroid-containing preformulation is increased by less than 30% compared with the particle size of the excipient.

6. Process for producing homogeneous preformulations which contain steroids in high concentration according to Claim 5, **characterized in that** the pharmaceutically customary excipient is present in micronized form.

## Revendications

1. Préformulations homogènes contenant des stéroïdes à forte concentration, pour la production de préparations pharmaceutiques solides et semi-solides à faibles doses, ayant une teneur en stéroïde de 0,001 à 1% en poids, pouvant être obtenues par
évaporation du dispersant contenant la substance active stéroïdienne, à partir d'une suspension avec un adjuvant pharmaceutiquement usuel dispersé,
en produisant un nébulisat dont le diamètre moyen de gouttes est supérieur au diamètre moyen des particules d'adjuvant
et la taille maximale des particules séchées de la préformulation contenant un stéroïde est augmentée de moins de 30% par rapport à la taille de particule de l'adjuvant.

2. Préformulations homogènes contenant des stéroïdes à forte concentration, pour la production de préparations pharmaceutiques solides et semi-solides à faibles doses selon la revendication 1, **caractérisées en ce que** la concentration de la substance active stéroïdienne va de 0,001 à 50% en poids.

3. Préformulations homogènes contenant des stéroïdes à forte concentration selon les revendications 1 et 2, **caractérisées en ce que** l'adjuvant pharmaceutiquement usuel se trouve sous forme micronisée.

4. Préformulations homogènes contenant des stéroïdes à forte concentration selon les revendications 1 à 3, **caractérisées en ce que** la substance active stéroïdienne représente au moins un composant choisi dans le groupe des hormones sexuelles.

5. Procédé pour la production de préformulations homogènes contenant des stéroïdes à forte concentration, pour des préparations pharmaceutiques solides et semi-solides à faibles doses, ayant une teneur en stéroïde de 0,001 à 1 % en poids, **caractérisé en ce qu'**on
évapore le dispersant contenant la substance active stéroïdienne, à partir d'une suspension avec un adjuvant pharmaceutiquement usuel dispersé,
en produisant un nébulisat dont le diamètre moyen de gouttes est supérieur au diamètre moyen des particules d'adjuvant
et la taille maximale des particules séchées de la préformulation contenant un stéroïde est augmentée de moins de 30 % par rapport à la taille de particule de l'adjuvant.

6. Procédé pour la production de préformulations homogènes contenant des stéroïdes à forte concentration selon la revendication 5, **caractérisé en ce que** l'adjuvant pharmaceutiquement usuel se trouve sous forme micronisée.
